(19) Europäisches Patentamt
European Patent Office
Office européen des brevets

(11) EP 4 769 417 A1

(12) **EUROPEAN PATENT APPLICATION**
published in accordance with Art. 153(4) EPC

(43) Date of publication:
**01.07.2026 Bulletin 2026/27**

(21) Application number: **24859563.9**

(22) Date of filing: **21.08.2024**

(51) International Patent Classification (IPC):
***G16C 10/00*** *(2019.01)*

(52) Cooperative Patent Classification (CPC):
**G16C 10/00**

(86) International application number:
**PCT/JP2024/029599**

(87) International publication number:
**WO 2025/047530 (06.03.2025 Gazette 2025/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **25.08.2023 JP 2023137135**

(71) Applicant: **Resonac Corporation**
**Tokyo 105-7325 (JP)**

(72) Inventors:
- **SAITO, Tatsushi**
**Tokyo 105-7325 (JP)**
- **NISHIZAWA, Shohei**
**Tokyo 105-7325 (JP)**
- **OCHIAI, Kohei**
**Tokyo 105-7325 (JP)**

(74) Representative: **Strehl & Partner mbB**
**Maximilianstrasse 54**
**80538 München (DE)**

# (54) INFORMATION PROCESSING DEVICE, INFORMATION PROCESSING METHOD, AND PROGRAM

(57) A molecular dynamics calculation unit configured to calculate a motion of a molecular group including at least one of atoms or molecules, by using molecular dynamics calculation; a mean square displacement calculation unit configured to calculate a mean square displacement for each of the atoms and the molecules; a diffusion coefficient calculation unit configured to calculate a diffusion coefficient for each of the atoms and the molecules based on the mean square displacement for each of the atoms and the molecules; a fitting unit to configured perform fitting on a cumulative probability of the diffusion coefficient with a cumulative distribution function; and a diffusion coefficient determination unit configured to determine a diffusion coefficient of the molecular group, based on the cumulative distribution function used for the fitting, are provided.

FIG.7

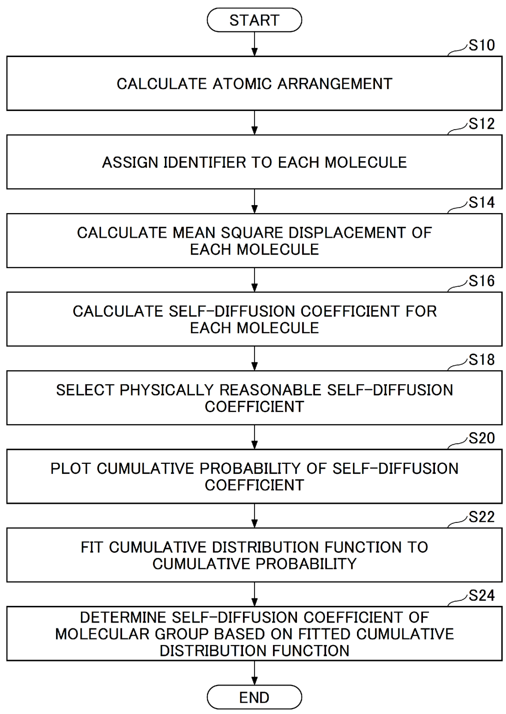




Processed by Luminess, 75001 PARIS (FR)

## Description

### TECHNICAL FIELD

The present disclosure relates to an information processing apparatus, an information processing method, and a program.

### BACKGROUND ART

For example, as one of the methods for obtaining the diffusion coefficient, conventionally, there has been known a method in which the motion of atoms and molecules is calculated by using molecular dynamics calculation, the mean square displacement of individual atoms and molecules at each time is obtained, the mean square displacement of individual atoms and molecules is averaged at each time, and the slope of the averaged mean square displacement with respect to time is calculated as the diffusion coefficient.

Patent Document 1 discloses a technique for obtaining the diffusion coefficient by using molecular dynamics calculation.

### CITATION LIST

### PATENT DOCUMENT

Patent document 1: Japanese Patent No. 3309820

### SUMMARY OF INVENTION

### TECHNICAL PROBLEM

In the molecular dynamics calculation, the distance moved by each atom and molecule is different due to the characteristics of the method. Therefore, in the molecular dynamics calculation, the number and degree of atoms and molecules that move greatly tend to vary from trial to trial. In the conventional method for obtaining the diffusion coefficient, the atoms and molecules that move greatly contribute more to the diffusion coefficient, and the atoms and molecules that move greatly cause the diffusion coefficient to be overestimated or scattered.

On the other hand, it is not preferable to arbitrarily ignore atoms and molecules that have moved greatly in a conventional method for determining diffusion coefficients, because atoms and molecules that have moved greatly are also elements that express diffusion phenomena.

An object of the present disclosure is to provide an information processing apparatus, an information processing method, and a program that improve the accuracy of a diffusion coefficient of a molecular group calculated by using molecular dynamics calculation.

### SOLUTION TO PROBLEM

The present disclosure has the following configurations.

[1] An information processing apparatus including:

> a molecular dynamics calculation unit configured to calculate a motion of a molecular group including at least one of atoms or molecules, by using molecular dynamics calculation;
> a mean square displacement calculation unit configured to calculate a mean square displacement for each of the atoms and the molecules;
> a diffusion coefficient calculation unit configured to calculate a diffusion coefficient for each of the atoms and the molecules based on the mean square displacement for each of the atoms and the molecules;
> a fitting unit configured to perform fitting on a cumulative probability of the diffusion coefficient with a cumulative distribution function; and
> a diffusion coefficient determination unit configured to determine a diffusion coefficient of the molecular group, based on the cumulative distribution function used for the fitting.

[2] The information processing apparatus according to [1], wherein the diffusion coefficient determination unit determines a representative value of the diffusion coefficient of the molecular group, by using the cumulative

distribution function used for the fitting.

[3] The information processing apparatus according to [1] or [2], wherein the cumulative distribution function is an error function or a complementary error function.

[4] The information processing apparatus according to any one of [1] to [3], wherein the diffusion coefficient of the molecular group is a diffusion coefficient of a polymer group that diffuses on a surface.

[5] The information processing apparatus according to any one of [1] to [4], wherein the molecular group is a lubricant molecular group forming a lubricating layer of a magnetic recording medium.

[6] An information processing method executed by an information processing apparatus, the information processing method including:

a molecular dynamics calculation procedure of calculating a motion of a molecular group including at least one of atoms or molecules, by using molecular dynamics calculation;
a mean square displacement calculation procedure of calculating a mean square displacement for each of the atoms and the molecules;
a diffusion coefficient calculation procedure of calculating a diffusion coefficient for each of the atoms and the molecules based on the mean square displacement for each of the atoms and the molecules;
a fitting procedure of performing fitting on a cumulative probability of the diffusion coefficient with a cumulative distribution function; and
a diffusion coefficient determination procedure of determining a diffusion coefficient of the molecular group, based on the cumulative distribution function used for the fitting.

[7] A program that causes an information processing apparatus to execute:

a molecular dynamics calculation step of calculating a motion of a molecular group including at least one of atoms or molecules, by using molecular dynamics calculation;
a mean square displacement calculation step of calculating a mean square displacement for each of the atoms and the molecules;
a diffusion coefficient calculation step of calculating a diffusion coefficient for each of the atoms and the molecules based on the mean square displacement for each of the atoms and the molecules;
a fitting step of performing fitting on a cumulative probability of the diffusion coefficient with a cumulative distribution function; and
a diffusion coefficient determination step of determining a diffusion coefficient of the molecular group, based on the cumulative distribution function used for the fitting.

ADVANTAGEOUS EFFECTS OF INVENTION

According to the present disclosure, it is possible to provide an information processing apparatus, an information processing method, and a program for improving the accuracy of the diffusion coefficient of the molecular group calculated by using molecular dynamics calculation.

BRIEF DESCRIPTION OF DRAWINGS

[FIG. 1] FIG. 1 is a configuration diagram of an example of an information processing system according to the present embodiment.
[FIG. 2] FIG. 2 is a hardware configuration diagram of an example of a computer according to the present embodiment.
[FIG. 3] FIG. 3 is a graph illustrating the mean square displacement of each molecule using a lubricant molecule as an example.
[FIG. 4A] FIG. 4A is an explanatory view of an example of a self-diffusion coefficient obtained by a general method.
[FIG. 4B] FIG. 4B is an explanatory view of an example of a self-diffusion coefficient obtained by a general method.
[FIG. 5A] FIG. 5A is an explanatory view of an example of a method according to the present embodiment for obtaining a diffusion coefficient.
[FIG. 5B] FIG. 5B is an explanatory view of an example of a method according to the present embodiment for obtaining a diffusion coefficient.
[FIG. 6] FIG. 6 is a functional configuration diagram of an example of an information processing system according to the present embodiment.
[FIG. 7] FIG. 7 is a flowchart illustrating an example of a processing procedure of an information processing system

according to the present embodiment.

DESCRIPTION OF EMBODIMENTS

Next, an embodiment of the present invention will be described in detail. The present invention is not limited to the following embodiments.

[First embodiment]

<System configuration>

FIG. 1 is a configuration diagram of an example of an information processing system 1 according to the present embodiment. The information processing system 1 of FIG. 1 includes a server apparatus 10 and a client terminal 12. The server apparatus 10 and the client terminal 12 of the information processing system 1 are connected to each other via a communication network 18 such as a local area network (LAN) or the Internet so as to enable data communication.

The client terminal 12 is an information processing apparatus operated by an operator such as a PC, a tablet terminal, or a smartphone. The client terminal 12 displays a screen for receiving input of information from the operator on a display device and receives input of information from the operator. The client terminal 12 transmits a processing request to the server apparatus 10 according to input by the operator and causes the server apparatus 10 to execute the processing. The client terminal 12 receives information on the execution result of the processing by the server apparatus 10, displays the information on a display device, and makes the operator confirm the information.

The server apparatus 10 is an information processing apparatus such as a PC or a workstation. The server apparatus 10 receives a processing request from the client terminal 12 and executes the processing. The server apparatus 10 transmits the processing result information to the client terminal 12, and makes the client terminal 12 display the processing result information.

The information processing system 1 may be implemented by the server apparatus 10 that provides the client terminal 12 with a program download function, and the client terminal 12 that executes the downloaded program. The client terminal 12 that has executed the program calculates a diffusion coefficient of a group of molecules as described below by using molecular dynamics calculation, and displays the diffusion coefficient on a display device, for example, to make the operator confirm the diffusion coefficient.

The molecular dynamics calculation is a technique for tracking the motion of atoms and molecules according to the laws of physics, and can calculate physical quantities related to the motion of atoms and molecules such as diffusion coefficients. Some physical quantities such as the diffusion coefficient may be greatly increased or decreased by a phenomenon (rare event) that rarely occurs.

The information processing system 1 may be implemented by the server apparatus 10 having a web server function and the client terminal 12 executing the web application by the web browser function. The client terminal 12 executing the web application by the web browser function displays, for example, on a display device, the diffusion coefficient of a molecular group calculated as described below by using molecular dynamics calculation by the server apparatus 10 having the web server function, and makes an operator confirm the diffusion coefficient.

The information processing system 1 may be implemented by having the client terminal 12 that executes a program and the server apparatus 10 perform the processing in cooperation with each other. By having the client terminal 12 that executes a program and the server apparatus 10 perform the processing in cooperation with each other, the diffusion coefficient of a molecular group is calculated as described below by using a molecular dynamics calculation, and the calculated diffusion coefficient of the molecular group is displayed, for example, on a display device, and operator is made to confirm the diffusion coefficient.

Note that the information processing system 1 illustrated in FIG. 1 is an example, and it is needless to say that there are various system configuration examples according to applications and purposes. For example, the server apparatus 10 may be implemented by a plurality of computers performing processing in cooperation, or may be implemented as a cloud computing service. The information processing system 1 may be implemented by a stand-alone computer.

<Hardware configuration>

The server apparatus 10 and the client terminal 12 illustrated in FIG. 1 are implemented by a computer 500 having a hardware configuration illustrated in FIG. 2, for example.

FIG. 2 is a hardware configuration diagram of an example of the computer 500 according to the present embodiment. The computer 500 illustrated in FIG. 2 includes an input device 501, a display device 502, an external I/F 503, a RAM (Random Access Memory) 504, a ROM (Read Only Memory) 505, a CPU (Central Processing Unit) 506, a

communication I/F 507, and an HDD (Hard Disk Drive) 508, etc., which are mutually connected via a bus B. The input device 501 and the display device 502 may be configured to be used upon being connected to the computer 500.

The input device 501 is a touch panel, operation keys and buttons, a keyboard, a mouse, or the like, which is used by an operator to input various signals. The display device 502 includes, for example, a liquid crystal or organic EL display for displaying a screen, and a speaker for outputting sound data such as voice or sound. The communication I/F 507 is an interface for the computer 500 to perform data communication.

The HDD 508 is an example of a nonvolatile storage device for storing programs and data. The stored programs and data include an OS, which is basic software for controlling the entire computer 500, and application programs for providing various functions on the OS. The computer 500 may use a drive device (such as a solid state drive (SSD)) using a flash memory as a storage medium instead of the HDD 508 or together with the HDD 508.

The external I/F 503 is an interface with an external device. The external device includes a recording medium 503a. Thus, the computer 500 can read and/or write to the recording medium 503a via the external I/F 503. The recording medium 503a includes a flexible disk, CD, DVD, SD memory card, USB memory, and the like.

The ROM 505 is an example of a nonvolatile semiconductor memory (storage device) that can hold programs and data even when the power is turned off. The ROM 505 stores programs and data such as BIOS, OS settings, and network settings that are executed when the computer 500 is started. The RAM 504 is an example of a volatile semiconductor memory (storage device) that temporarily holds programs and data.

The CPU 506 is an arithmetic unit that loads programs and data from storage devices such as the ROM 505 and the HDD 508 into the RAM 504 and executes processing, thereby implementing control and functions of the entire computer 500. The computer 500 according to the present embodiment can implement various functions of the server apparatus 10 and the client terminal 12 by executing programs.

A program installed in the RAM 504 is installed by reading a program recorded on the recording medium 503a through the external I/F 503, for example. Alternatively, the program may be installed by being downloaded from the communication network 18 via the communication I/F 507.

<Method for determining the diffusion coefficient>

Here, in order to facilitate understanding of the method for determining the diffusion coefficient according to the present embodiment, a description will be given by comparing with a general method for determining the diffusion coefficient. Hereinafter, atoms and molecules may simply be referred to as molecules by abbreviation.

In one of the general methods for determining the diffusion coefficient, the motion of a group of molecules is calculated by using molecular dynamics calculation, the mean square displacement ($u^2$) of each molecule at each time is obtained, the obtained mean square displacement is averaged among molecules, and the diffusion coefficient is calculated by using, for example, the following equation (1). Equation (1) is an example of an equation for calculating the self-diffusion coefficient. In equation (1), the slope of the averaged mean square displacement with respect to time is calculated as the self-diffusion coefficient.

[Equation 1]

$$\text{SELF-DIFFUSION COEFFICIENT } SDC = \frac{u^2}{6t} \qquad \cdots (1)$$

In the molecular dynamics calculation, due to the characteristics of the method, for example, as illustrated in FIG. 3, the mean square displacement of each molecule tends to vary. FIG. 3 is a graph illustrating the mean square displacement of each molecule by using a lubricant molecule as an example. The lubricant molecule is, for example, a compound represented by the following chemical formula (1). In the graph illustrated in FIG. 3, an average line illustrating the average mean square displacement of each molecule is added. As illustrated in FIG. 3, the mean square displacement of the molecules included in the molecular group differs from molecule to molecule. For example, in FIG. 3, the mean square displacement of approximately 1/3 of the total molecules greatly exceeds the average line.

[Chemical formula 1]

$$R1-CF_2-CF_2-O-\left(CF_2-CF_2-CF_2-O\right)_6-CF_2-CF_2-R2 \quad \cdots (1)$$

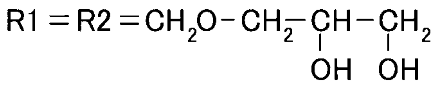

FIG. 4 is a diagram of an example of self-diffusion coefficients obtained by the general method. The self-diffusion coefficients of the respective molecules obtained by the general method vary as illustrated in FIG. 4A. The distribution of the self-diffusion coefficients of the respective molecules in FIG. 4A obtained by the general method is, for example, as illustrated in FIG. 4B.

FIG. 4B illustrates a distribution of the self-diffusion coefficients of molecules obtained by the general method and an average line indicating the self-diffusion coefficients of a group of molecules. As illustrated in FIG. 4B, the self-diffusion coefficients of a group of molecules obtained by the general method are greatly contributed by molecules (rare molecules) whose self-diffusion coefficients greatly exceed the average illustrated in the region 1000 of FIG. 4A, and the greatly moved molecules cause the diffusion coefficients of the group of molecules to be overestimated or to vary.

Therefore, in the method according to the present embodiment for obtaining the diffusion coefficients, the motion of a group of molecules is calculated by using molecular dynamics calculation, the mean square displacement of each molecule at each time is obtained, and the self-diffusion coefficients of the respective molecules are calculated by using the equation (1) based on the obtained mean square displacement of each molecule. In the method according to the present embodiment for obtaining the diffusion coefficients, the calculated self-diffusion coefficients of the respective molecules are plotted, for example, as illustrated in FIG. 5A. FIGS. 5A and 5B are an explanatory diagram of an example of the method according to the present embodiment for obtaining the diffusion coefficients. FIG. 5A illustrates the cumulative probability on the vertical axis and the self-diffusion coefficient on the horizontal axis.

In the method according to the present embodiment for obtaining the diffusion coefficient, for example, as illustrated in FIG. 5A, the curve of the self-diffusion coefficient of each molecule obtained is regressed by the cumulative distribution function, and the self-diffusion coefficient of the molecular group is calculated based on the regressed cumulative distribution function.

FIG. 5B illustrates the distribution of the self-diffusion coefficient of the molecule obtained by the method according to the present embodiment for obtaining the diffusion coefficient and the mean line indicating the self-diffusion coefficient of the molecular group. FIG. 5B illustrates a Gaussian distribution obtained by differentiating the cumulative distribution function of FIG. 5A.

As illustrated in FIG. 5B, in the method according to the present embodiment for obtaining the diffusion coefficient, the accuracy of the mean line indicating the self-diffusion coefficient of the molecular group is improved compared with the mean line indicating the self-diffusion coefficient of the molecular group of FIG. 4B obtained by the general method. As described above, according to the method according to the present embodiment for obtaining the diffusion coefficient, the diffusion coefficient of the molecular group (representative value of the diffusion coefficient) can be calculated while suppressing the variation of the motion of each molecule.

<Functional configuration>

The functional configuration of the information processing system 1 according to the present embodiment will be described. FIG. 6 is a functional configuration diagram of an example of the information processing system 1 according to the present embodiment. In the configuration diagram of FIG. 6, portions unnecessary for the description of the present embodiment are appropriately omitted.

The server apparatus 10 of the information processing system 1 illustrated in FIG. 6 includes a request reception unit 20, a response transmission unit 22, a molecular dynamics calculation unit 24, a mean square displacement calculation unit 26, a diffusion coefficient calculation unit 28, a fitting unit 30, a diffusion coefficient determination unit 32, a display control unit 34, and a storage unit 40. The client terminal 12 of the information processing system 1 has an information display unit 50, an operation reception unit 52, a request transmission unit 54, and a response reception unit 56.

The information display unit 50 displays, on a display device 502, a screen for receiving input of information from an operator and information on the execution result of processing by the server apparatus 10. The operation reception unit

52 receives operation by an operator such as input of information. The request transmission unit 54 transmits a processing request corresponding to the input of information from the operator, to the server apparatus 10. The response reception unit 56 receives a response to the processing request transmitted by the request transmission unit 54, from the server apparatus 10.

The request reception unit 20 receives the processing request from the client terminal 12. The response transmission part 22 responds by transmitting the execution result of the processing corresponding to the processing request, to the client terminal 12. The molecular dynamics calculation unit 24 calculates the motion of each individual molecule included in the molecular group by using molecular dynamics calculation. The mean square displacement calculation unit 26 calculates the mean square displacement of each individual molecule.

The diffusion coefficient calculation unit 28 calculates the diffusion coefficient of each individual molecule based on the calculated mean square displacement of each individual molecule. The fitting unit 30 fits the cumulative probability of the calculated diffusion coefficient of each individual molecule with a cumulative distribution function. The fitting unit 30 plots, for example, the cumulative probability of the calculated diffusion coefficient for each molecule as illustrated in FIG. 5A. The fitting unit 30 regresses the obtained curve of the diffusion coefficient for each molecule as illustrated in FIG. 5A, for example, with the cumulative distribution function.

The diffusion coefficient determination unit 32 determines the diffusion coefficient of the molecular group based on the fitted cumulative distribution function. The diffusion coefficient determination unit 32 determines the diffusion coefficient of the molecular group based on the regressed cumulative distribution function as illustrated in FIG. 5A, for example. The display control unit 34 controls the display of the client terminal 12. The storage unit 40 stores programs and data necessary for processing executed by the server apparatus 10 or the client terminal 12.

Note that the functional configuration illustrated in FIG. 6 is an example, and various configurations can be implemented. For example, the storage unit 40 may include a storage device, a computer, or a cloud storage capable of performing data communication with the server apparatus 10 and the client terminal 12 via, for example, the communication network 18.

<Processing>

In the following, as an example of a method for obtaining the diffusion coefficient according to the present embodiment, an example of calculating the self-diffusion coefficient of a group of lubricant molecules constituting a lubricating layer of a magnetic recording medium, by using molecular dynamics calculation, will be described. For example, a lubricating layer of a magnetic recording medium is formed by applying a lubricant to the surface of a protective layer formed by using carbon or the like on the magnetic recording layer.

For example, in order to simulate a lubricating layer of a magnetic recording medium, a structural model of the lubricant molecules (hereinafter referred to as the lubricant molecular model) and a structural model of the protective layer (hereinafter referred to as protective layer model) are prepared, and by laying an arbitrary number of lubricant molecule models on the protective layer model, a situation in which a lubricating layer is formed on the protective layer can be reproduced.

FIG. 7 is a flowchart illustrating an example of a processing procedure of the information processing system 1 according to the present embodiment. In step S10, the molecular dynamics calculation unit 24 calculates the following atomic arrangement. The molecular dynamics calculation unit 24 constructs a lubricant molecule constituting the lubricating layer and a protective layer model. Examples of lubricant molecules used in a magnetic recording medium include compounds having a polar group such as a hydroxy group at the terminal of a fluoropolymer having a repeating structure including a perfluoro ether chain ($-CF_2-$). The molecular mass thereof is, for example, approximately 1000 to 10,000. For example, the following chemical formula (2) represents an example of a lubricant molecule calculated in the present embodiment. The polymer of chemical formula (2) is a chain polymer. Alternatively, the polymer may be chemical formula (3).

R1-X-R2 ... (2)

R1-X-R3-X-R2 ... (3)

In chemical formulas (2) and (3), X is a main chain portion including a repeating structure. R1 and R2 are a first terminal portion and a second terminal portion, respectively, and have at least one polar group represented by a hydroxyl group. The structures of R1 and R2 may be the same or different. R3 is a connecting part. R3 has the same characteristics as R1 and R2.

The structure of the above molecule is optimized by using quantum chemical calculation, and a lubricant molecular model is obtained. This lubricant molecular model can be appropriately used in molecular dynamics calculation because it has a molecular structure and information of the charge of each atom.

The protective layer is a layer for protecting the recording layer. The protective layer is composed of carbon atoms or silicon carbide. As the unit structure of the layer composed of carbon atoms, a unit structure of graphene or diamond carbon can be used. The graphene or diamond carbon structure is preferably doped with oxygen or nitrogen. By using these as adsorption sites, it is possible to strengthen the bond with the polar group of the lubricant. For example, the "unit structure" of the protective layer of the graphene structure is the smallest unit composed of six carbons. The protective layer may be composed of the thickness of one atomic layer or a plurality of atomic layers.

The protective layer model is obtained by optimizing the unit structure of such a protective layer by quantum chemical calculation, and repeating the optimized unit structure in the plane direction within a periodic boundary cell used in molecular dynamics calculation described below. This protective layer model can be appropriately used in molecular dynamics calculation because it has information on the structure and charge of each atom.

The initial model of the lubricating layer (lubricating layer initial model) is constructed by using the lubricant molecular model and the protective layer model prepared as described above. The lubricating layer initial model for carrying out the simulation of the lubricating layer of the magnetic recording medium may be constructed so long as the lubricant molecular model is uniformly arranged on the protective layer model to some extent.

The lubricating layer initial model is preferably constructed in a periodic boundary cell having a size sufficient to arrange approximately 10 to 1000 lubricant molecules. As a method for arranging the lubricant molecules in the lubricating layer initial model, the lubricant molecules may be arranged near the protective layer model so as not to overlap each other, or a stabilized initial arrangement may be prepared by using molecular dynamics calculation or the like.

In step S12, the molecular dynamics calculation unit 24 assigns an identifier to each lubricant molecule of the lubricating layer initial model. In step S14, the molecular dynamics calculation unit 24 executes molecular dynamics calculation by using the constructed lubricating layer initial model. The mean square displacement calculation unit 26 calculates the mean square displacement of each lubricant molecule of the lubricating layer initial model from the result of the molecular dynamics calculation.

For example, the molecular dynamics calculation can be executed under the following conditions. The various conditions of the molecular dynamics calculation are not limited to the following.

- Cell size: 96Å×84Å×150Å (periodic boundary cell)
- Tracking time: 6 ns (time of 1 step: 2 fs, total number of steps: 3 million steps)
- Temperature: 300 K (assuming room temperature)
- Molecular force field: GAFF force field
- Method of calculating Coulomb interaction:
  Particle-Particle Particle-Mesh Ewald method

In step S16, the diffusion coefficient calculation unit 28 calculates the self-diffusion coefficient for each lubricant molecule based on the mean square displacement for each lubricant molecule in the lubricating layer initial model calculated from the result of the molecular dynamics calculation at the tracking time T by using the following equation (2).
[Equation 2]

$$SDC = \frac{1}{6T}\langle |r(T+t_0) - r(t_0)|^2 \rangle \qquad \cdots (2)$$

Here, the notation of $t_0$ is an initial time. The notation of r(t) is the position of the center of gravity of the lubricant molecule at time t.

In step S18, the diffusion coefficient calculating unit 28 selects a physically reasonable self-diffusion coefficient from the self-diffusion coefficients of the respective lubricant molecules calculated in step S16. The process of step S18 is a process of excluding the self-diffusion coefficients of molecules that have moved in such a manner that the self-diffusion coefficients cannot be easily obtained. For example, if the curve of the mean square displacement is mountainous, the slope of the regression line becomes close to 0 or negative, resulting in a physically unreasonable self-diffusion coefficient. Therefore, the diffusion coefficient calculating unit 28 selects a physically reasonable self-diffusion coefficient by setting a lower limit to the value of the self-diffusion coefficient to be used in the processes from step S20 and thereafter.

In step S20, the fitting unit 30 plots, for example, as illustrated in FIG. 5A, the cumulative probability of the self-diffusion coefficient of each lubricant molecule selected as the physically reasonable self-diffusion coefficient in step S18.

In step S22, the fitting unit 30 selects and fits an appropriate cumulative distribution function to the cumulative probability of the self-diffusion coefficient plotted in step S20. For example, the fitting unit 30 fits ln (SDC) of the self-diffusion coefficient of each lubricant molecule based on an error function or a complementary error function.

[Equation 3]

$$\text{ERROR FUNCTION } (\mathrm{erf}(x)) : \mathrm{erf}(x) = \frac{2}{\sqrt{\pi}} \times \int_0^x exp(-t^2)$$

$$\text{COMPLEMENTARY ERROR FUNCTION } (\mathrm{erfc}(x)) : \mathrm{erfc}(x) = 1 - \mathrm{erf}(x)$$

In step S24, the diffusion coefficient determination unit 32 determines the diffusion coefficient of the lubricant molecular group based on the cumulative distribution function fitted in step S22. For example, the diffusion coefficient determination unit 32 can determine the diffusion coefficient of the lubricant molecular group based on the cumulative distribution function regressed as illustrated in FIG. 5A.

The lubricity value measured in the experiment, and the self-diffusion coefficient of the lubricant molecular group in the lubricating layer calculated by the method according to the present embodiment for obtaining the diffusion coefficient, are correlated. Therefore, the self-diffusion coefficient of the lubricant molecular group in the lubricating layer calculated by the method according to the present embodiment can be used as an index of the degree of lubrication of the lubricating layer. Specifically, for molecules whose lubricity value is measured in the experiment, the lubricity of the lubricating layer can be relatively evaluated based on the self-diffusion coefficient of the lubricant molecular group in the lubricating layer calculated by the method according to the present embodiment.

Therefore, in the information processing system 1 according to the present embodiment, the performance of the lubricating layer can be evaluated based on the self-diffusion coefficient of the lubricant molecular group in the lubricating layer calculated by the method according to the present embodiment.

As described above, according to the information processing system 1 according to the present embodiment, it is possible to provide an information processing apparatus, an information processing method, and a program for improving the accuracy of the diffusion coefficient of a group of molecules calculated by using molecular dynamics calculation.

<Supplementary explanation>

In the present embodiment, the reason for fitting the cumulative probability instead of the distribution is that the regression curve cannot be uniquely determined if the distribution is used, and the cumulative probability is used in order to avoid this. To obtain the distribution, a range for obtaining the distribution of the diffusion coefficient is specified, and the number of divisions of the range is determined. Further, the number of molecules having the diffusion coefficient in the range is counted for each region obtained by the division, and is used as a data point. At this time, the range specification and the division method can be performed by any method. These setting methods can change the shape of the distribution and consequently affect the regression curve.

The cumulative distribution function is a "probability that a random variable has a value less than or equal to a certain value (x)". Therefore, the cumulative distribution function can be determined to be any function system according to the problem. In the present embodiment, the cumulative probability, plotted by taking the logarithm of the diffusion coefficient, is regressed based on the error function (or complementary error function).

As an example of the diffusion coefficient analysis, a slab model of nitrogen-doped carbon was used to prepare a model in which approximately 30 to 50 molecules with a molecular mass of 1000 or more were randomly adsorbed on the surface. The molecular dynamics calculation was performed with the atomic positions of the carbon slab fixed, and the diffusion coefficient of the molecular group was obtained. As conditions of the molecular dynamics calculation, the interaction between atoms was described by the gaff force field, and the charge amount of each atom was determined by using quantum chemical calculation.

The temperature was 300 K, the time was 6 ns, and the time step was 2 fs. For 1 diffusion coefficient analysis, 3 molecular dynamics calculations were performed under the same conditions. Each trial of the molecular dynamics calculation was performed from step S10 to step S18, and the diffusion coefficients of the respective molecules obtained in each trial were collectively used in step S20 and thereafter.

The calculation in the above embodiment is a model in which the diffusion coefficients of the respective molecules are easy to vary. The calculation in the above embodiment is characterized in that the surface diffusion and the diffusion coefficient of a macromolecular group are obtained. In surface diffusion, it is known that molecules and atoms on the surface diffuse more than in body diffusion (diffusion in a solid).

In the calculation in the above embodiment, the polar groups of molecules are adsorbed on N atoms on the carbon layer, and the molecules diffuse by changing their adsorption positions. A plurality of polar groups exist in a molecule, and adjacent molecules interact with each other. Therefore, there are more diffusion modes than atoms and small molecules, and the diffusion coefficient of each molecule varies widely.

[Other embodiment]

The server apparatus 10 according to the present embodiment may be configured to perform the above processing by having a plurality of computers cooperate with each other. For example, the server apparatus 10 according to the present embodiment may be configured to perform the above processing in cooperation with the client terminal 12.

The diffusion coefficient of the molecular group calculated in the present embodiment can be used to control, for example, a manufacturing apparatus for generating a magnetic recording medium such as an HDD by specifying the lubricant molecular group constituting the lubricating layer of the magnetic recording medium.

The information processing system 1 according to the present embodiment can improve the accuracy of the diffusion coefficient of the molecular group calculated by using molecular dynamics calculation. The diffusion coefficient of the molecular group calculated with high accuracy by the information processing system 1 according to the present embodiment can be used to control, for example, a manufacturing apparatus for a magnetic recording medium.

Although the present embodiment has been described above, it will be understood that various changes in the form and details can be made without departing from the spirit and scope of the claims. Although the present invention has been described based on the embodiments, the present invention is not limited to the above embodiments, and various modifications are possible within the scope of the claims. The present application claims priority to Japanese Patent Application No. 2023-137135, filed with the Japan Patent Office on August 25, 2023, the entire contents of which are incorporated herein by reference.

REFERENCE SIGNS LIST

1 information processing system
10 server apparatus
12 client terminal
18 communication network
24 molecular dynamics calculation unit
26 mean square displacement calculation unit
28 diffusion coefficient calculation unit
30 fitting unit
32 diffusion coefficient determination unit

**Claims**

**1.** An information processing apparatus comprising:

a molecular dynamics calculation unit configured to calculate a motion of a molecular group including at least one of atoms or molecules, by using molecular dynamics calculation;
a mean square displacement calculation unit configured to calculate a mean square displacement for each of the atoms and the molecules;
a diffusion coefficient calculation unit configured to calculate a diffusion coefficient for each of the atoms and the molecules based on the mean square displacement for each of the atoms and the molecules;
a fitting unit configured to perform fitting on a cumulative probability of the diffusion coefficient with a cumulative distribution function; and
a diffusion coefficient determination unit configured to determine a diffusion coefficient of the molecular group, based on the cumulative distribution function used for the fitting.

**2.** The information processing apparatus according to claim 1, wherein the diffusion coefficient determination unit determines a representative value of the diffusion coefficient of the molecular group, by using the cumulative distribution function used for the fitting.

**3.** The information processing apparatus according to claim 1 or 2, wherein the cumulative distribution function is an error function or a complementary error function.

**4.** The information processing apparatus according to any one of claims 1 to 3, wherein the diffusion coefficient of the molecular group is a diffusion coefficient of a polymer group that diffuses on a surface.

5. The information processing apparatus according to any one of claims 1 to 4, wherein the molecular group is a lubricant molecular group forming a lubricating layer of a magnetic recording medium.

6. An information processing method executed by an information processing apparatus, the information processing method comprising:

a molecular dynamics calculation procedure of calculating a motion of a molecular group including at least one of atoms or molecules, by using molecular dynamics calculation;
a mean square displacement calculation procedure of calculating a mean square displacement for each of the atoms and the molecules;
a diffusion coefficient calculation procedure of calculating a diffusion coefficient for each of the atoms and the molecules based on the mean square displacement for each of the atoms and the molecules;
a fitting procedure of performing fitting on a cumulative probability of the diffusion coefficient with a cumulative distribution function; and
a diffusion coefficient determination procedure of determining a diffusion coefficient of the molecular group, based on the cumulative distribution function used for the fitting.

7. A program that causes an information processing apparatus to execute:

a molecular dynamics calculation step of calculating a motion of a molecular group including at least one of atoms or molecules, by using molecular dynamics calculation;
a mean square displacement calculation step of calculating a mean square displacement for each of the atoms and the molecules;
a diffusion coefficient calculation step of calculating a diffusion coefficient for each of the atoms and the molecules based on the mean square displacement for each of the atoms and the molecules;
a fitting step of performing fitting on a cumulative probability of the diffusion coefficient with a cumulative distribution function; and
a diffusion coefficient determination step of determining a diffusion coefficient of the molecular group, based on the cumulative distribution function used for the fitting.

FIG.1

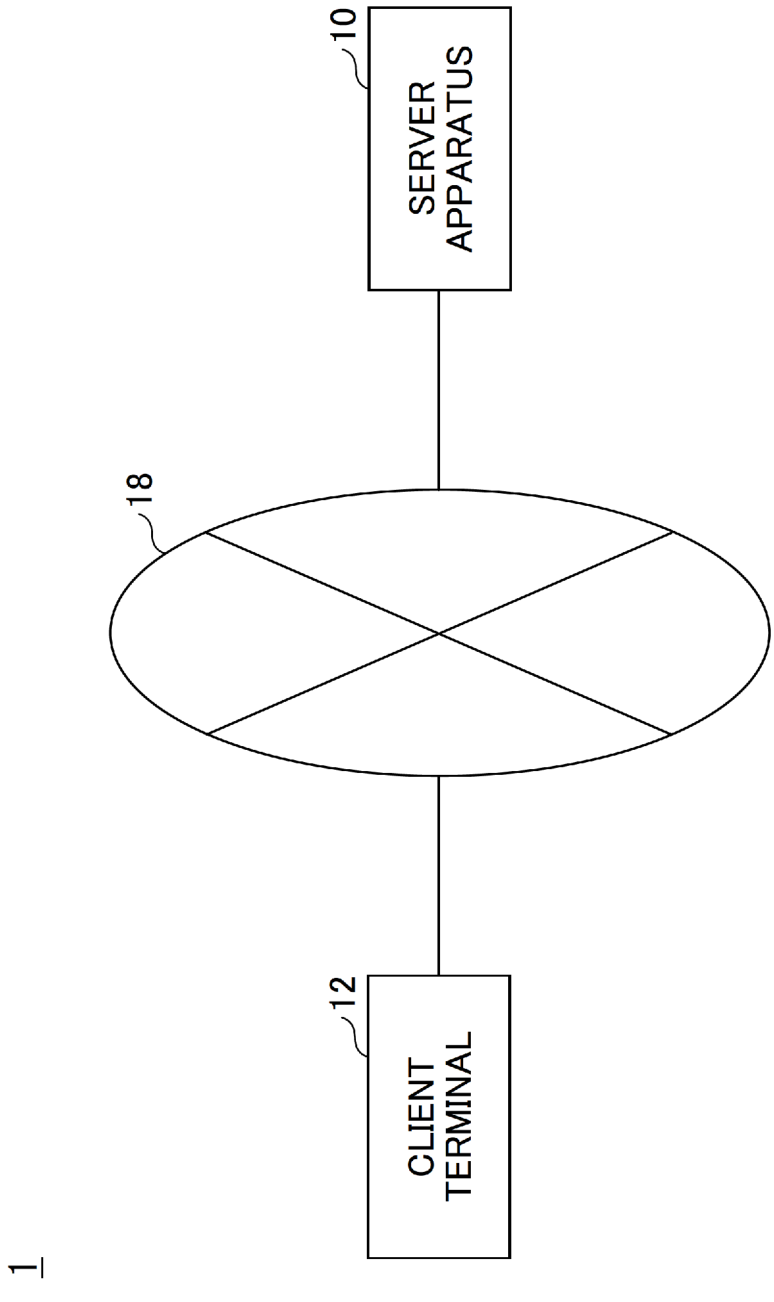

FIG.2

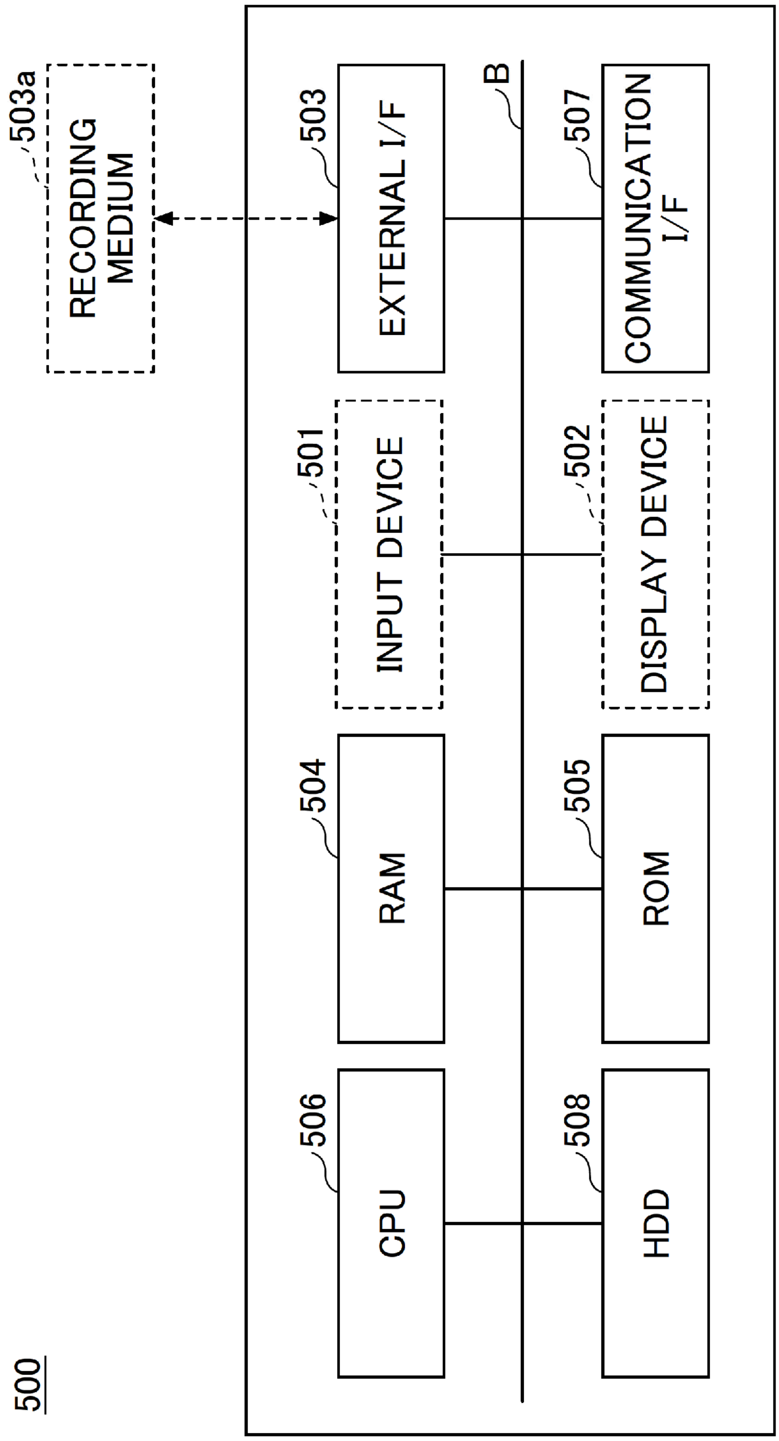
500
503a
RECORDING MEDIUM
503
EXTERNAL I/F
B
507
COMMUNICATION I/F
501
INPUT DEVICE
502
DISPLAY DEVICE
504
RAM
505
ROM
506
CPU
508
HDD

FIG.3

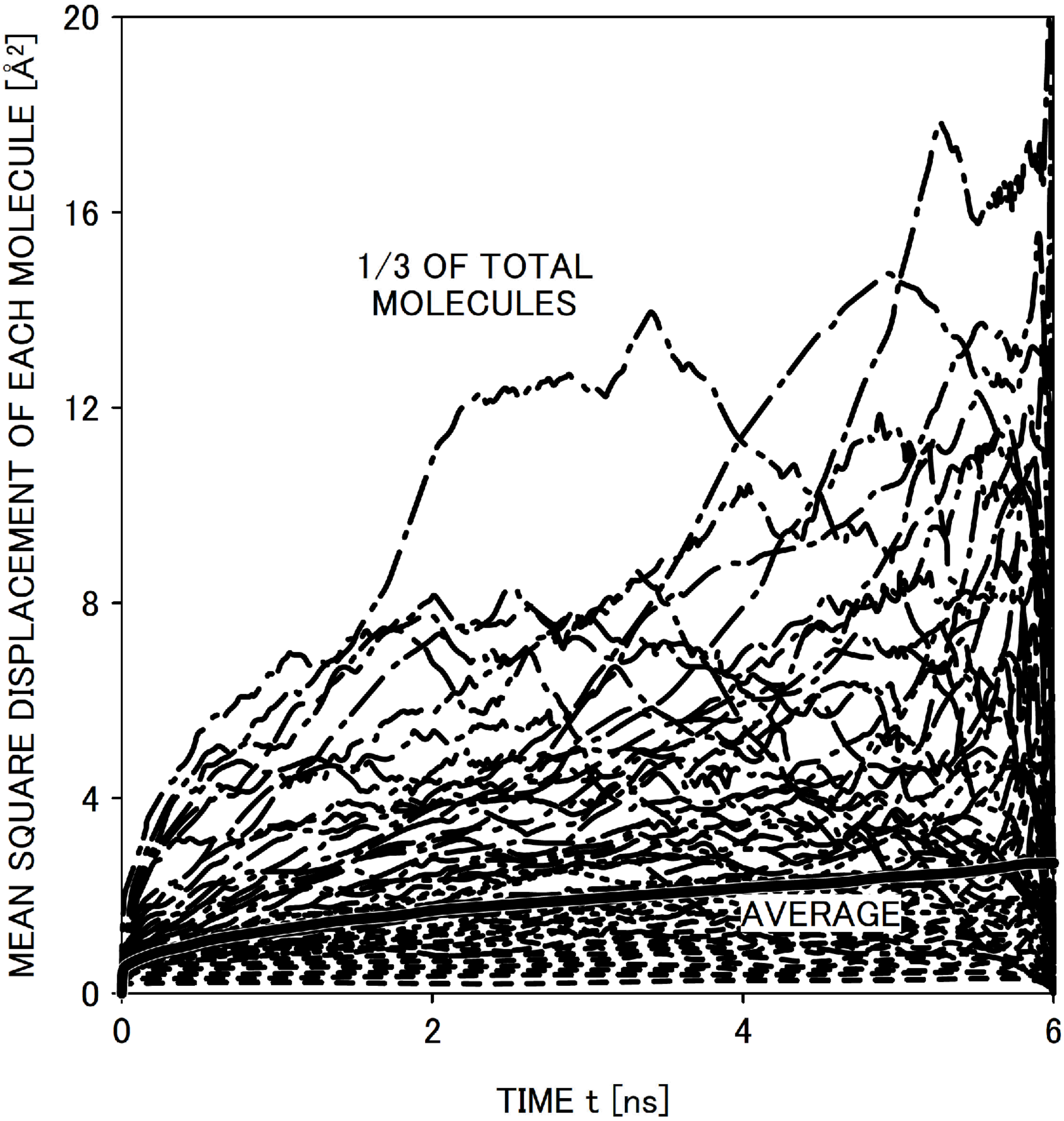
MEAN SQUARE DISPLACEMENT OF EACH MOLECULE [Å²]
20
16
12
8
4
0
1/3 OF TOTAL
MOLECULES
AVERAGE
0
2
4
6
TIME t [ns]

FIG.4A

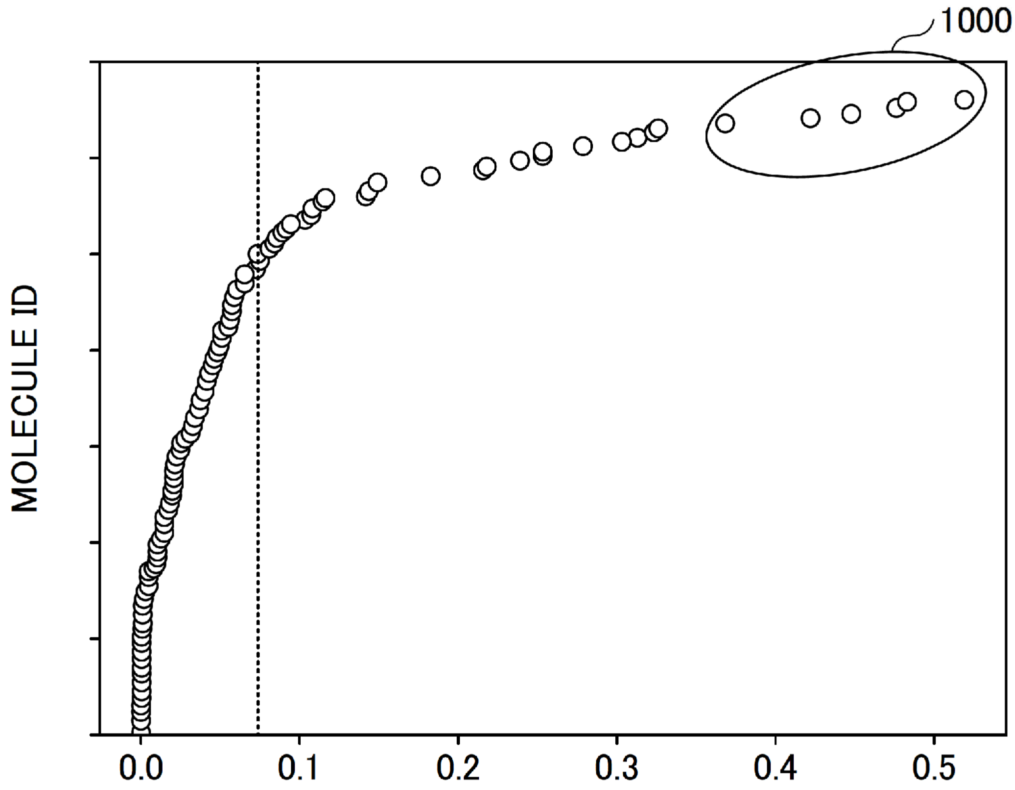


FIG.4B

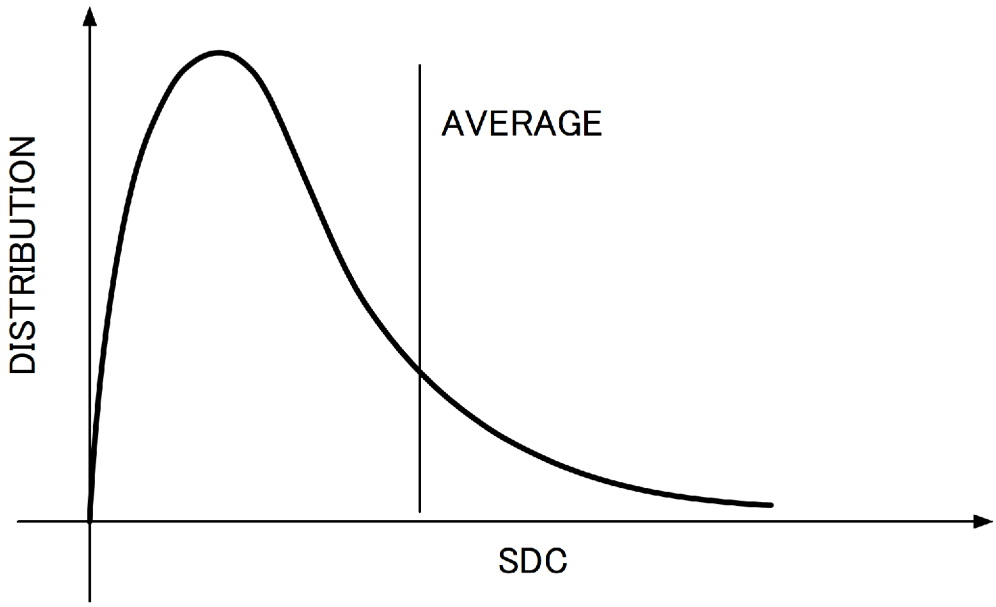

# FIG.5A

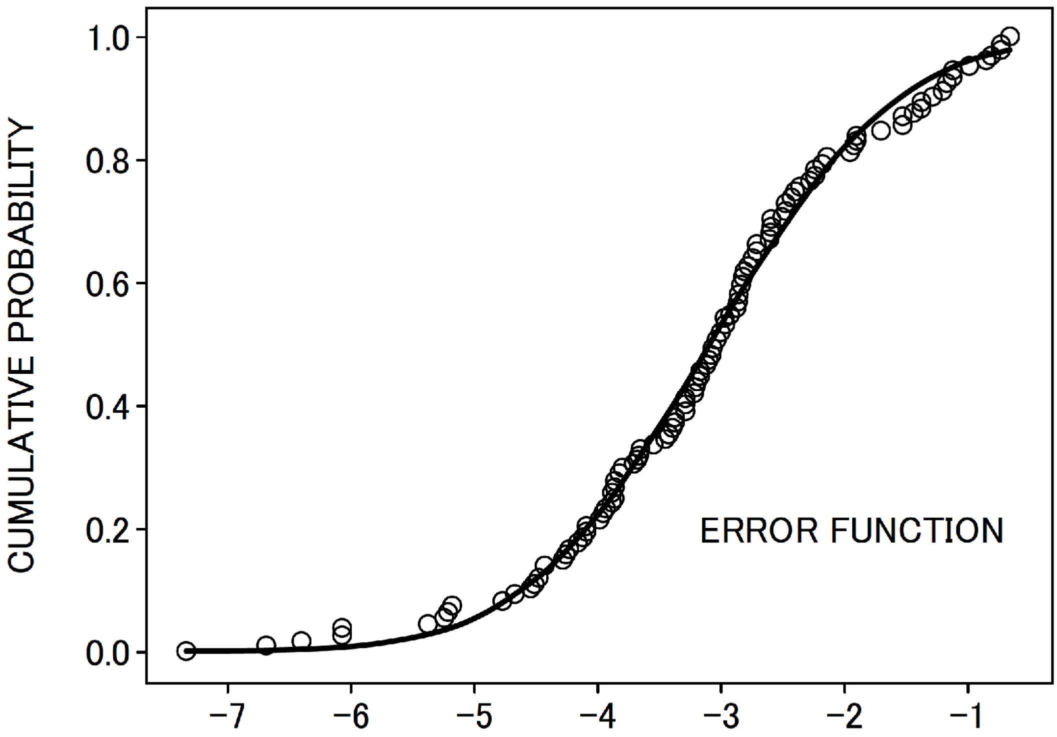
CUMULATIVE PROBABILITY
1.0
0.8
0.6
0.4
0.2
0.0
ERROR FUNCTION
−7
−6
−5
−4
−3
−2
−1

# FIG.5B

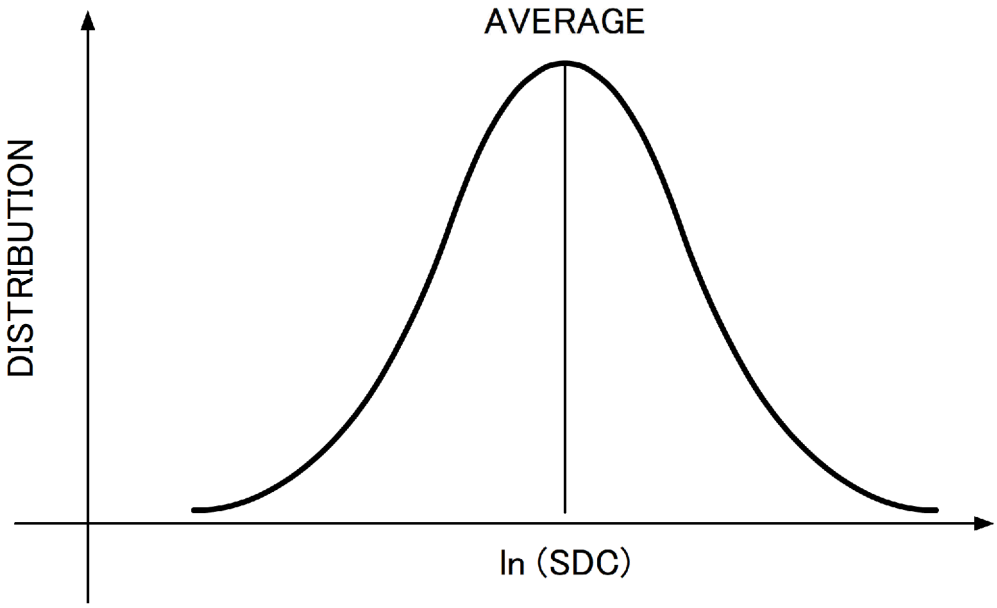
AVERAGE
DISTRIBUTION
ln (SDC)

FIG.6

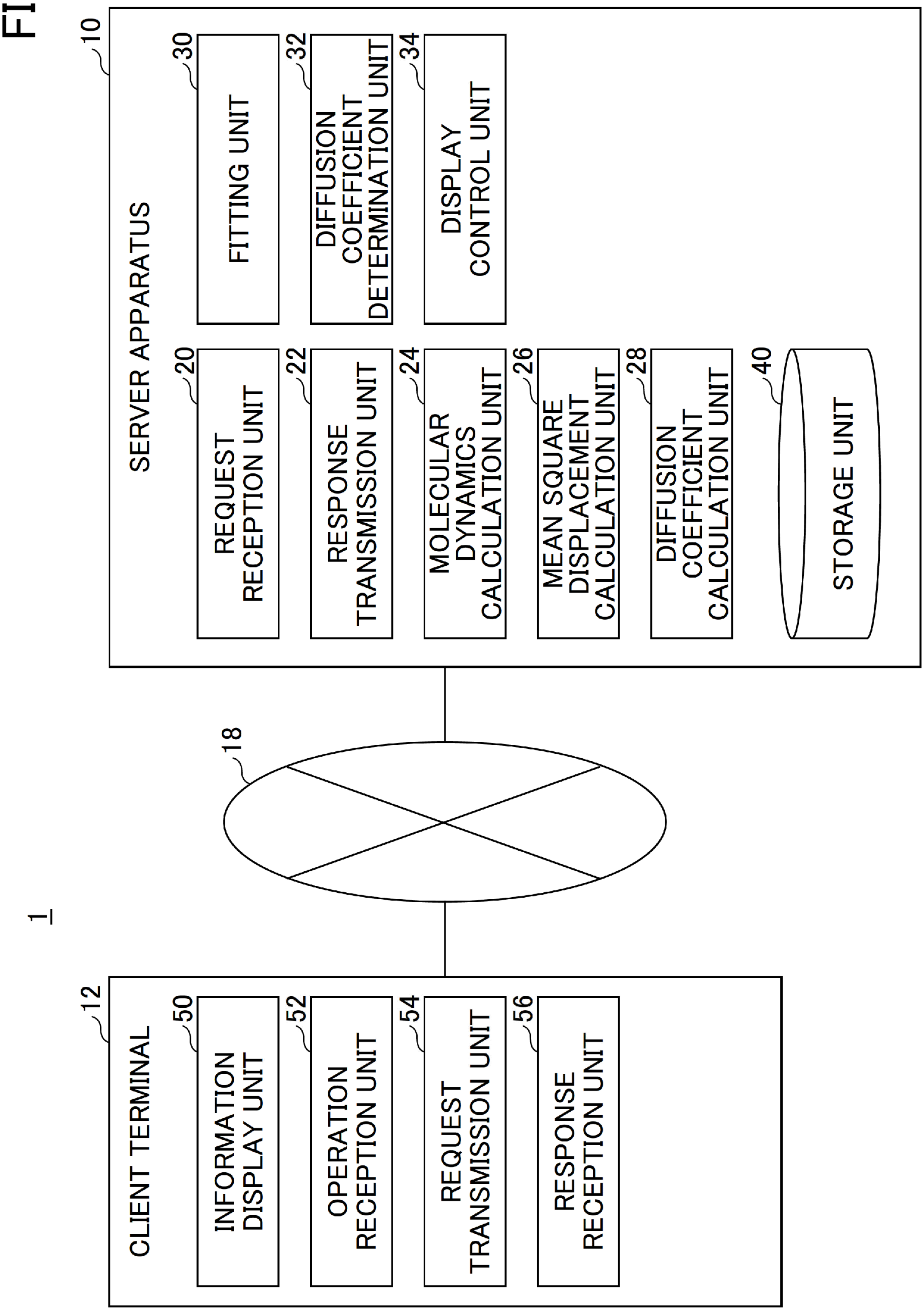
1
10
SERVER APPARATUS
20
REQUEST RECEPTION UNIT
22
RESPONSE TRANSMISSION UNIT
24
MOLECULAR DYNAMICS CALCULATION UNIT
26
MEAN SQUARE DISPLACEMENT CALCULATION UNIT
28
DIFFUSION COEFFICIENT CALCULATION UNIT
30
FITTING UNIT
32
DIFFUSION COEFFICIENT DETERMINATION UNIT
34
DISPLAY CONTROL UNIT
40
STORAGE UNIT
18
12
CLIENT TERMINAL
50
INFORMATION DISPLAY UNIT
52
OPERATION RECEPTION UNIT
54
REQUEST TRANSMISSION UNIT
56
RESPONSE RECEPTION UNIT

# FIG.7

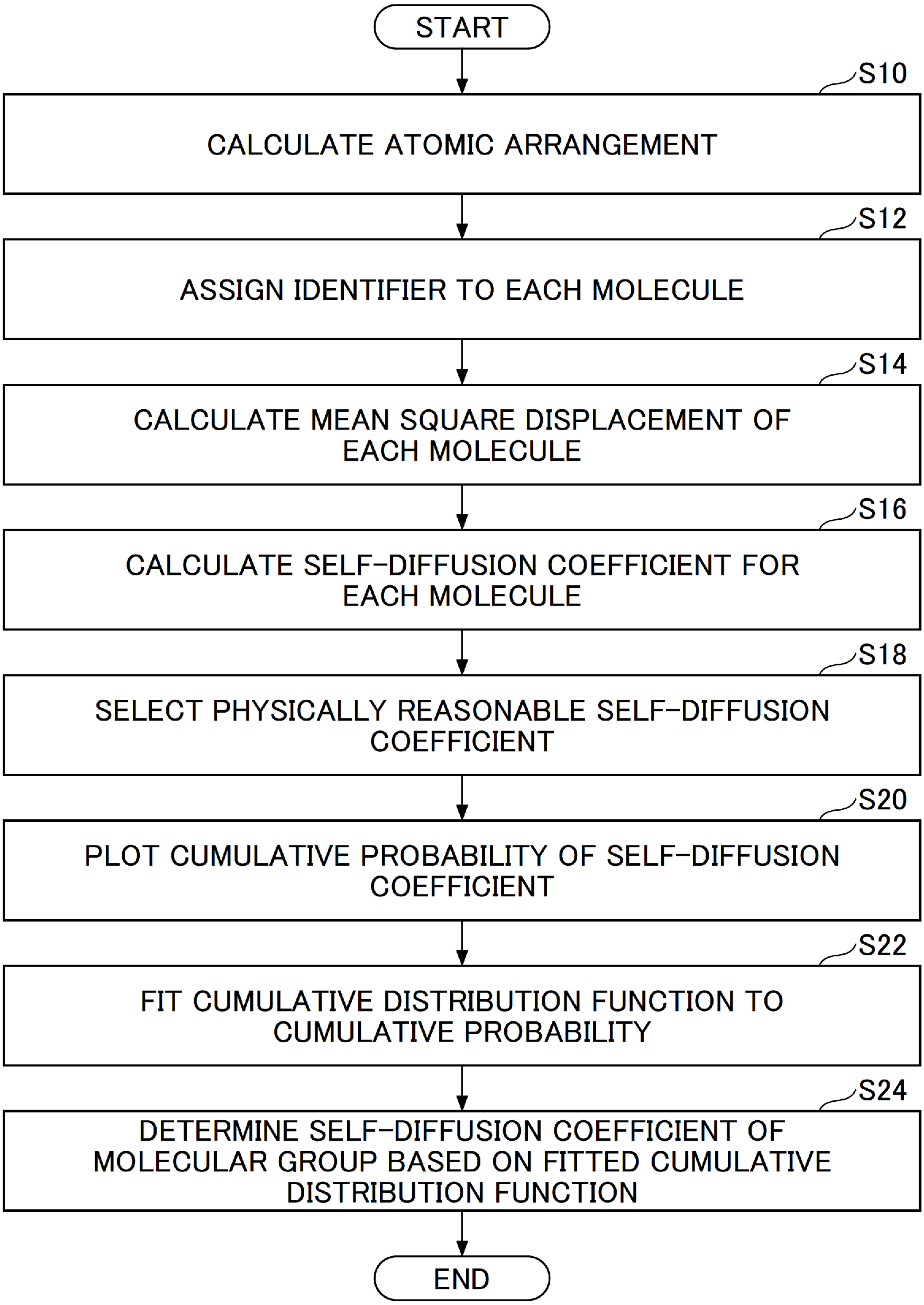
START
S10
CALCULATE ATOMIC ARRANGEMENT
S12
ASSIGN IDENTIFIER TO EACH MOLECULE
S14
CALCULATE MEAN SQUARE DISPLACEMENT OF EACH MOLECULE
S16
CALCULATE SELF-DIFFUSION COEFFICIENT FOR EACH MOLECULE
S18
SELECT PHYSICALLY REASONABLE SELF-DIFFUSION COEFFICIENT
S20
PLOT CUMULATIVE PROBABILITY OF SELF-DIFFUSION COEFFICIENT
S22
FIT CUMULATIVE DISTRIBUTION FUNCTION TO CUMULATIVE PROBABILITY
S24
DETERMINE SELF-DIFFUSION COEFFICIENT OF MOLECULAR GROUP BASED ON FITTED CUMULATIVE DISTRIBUTION FUNCTION
END

## INTERNATIONAL SEARCH REPORT

International application No.
**PCT/JP2024/029599**

**A. CLASSIFICATION OF SUBJECT MATTER**

***G16C 10/00***(2019.01)i
FI: G16C10/00

According to International Patent Classification (IPC) or to both national classification and IPC

**B. FIELDS SEARCHED**

Minimum documentation searched (classification system followed by classification symbols)

G16C10/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); IEEE Xplore

**C. DOCUMENTS CONSIDERED TO BE RELEVANT**

| Category* | Citation of document, with indication, where appropriate, of the relevant passages | Relevant to claim No. |
|---|---|---|
| A | CN 115544853 A (ZHEJIANG QIER ELECTROMECHANICAL TECHNOLOGY CO., LTD.) 30 December 2022 (2022-12-30) entire text, all drawings | 1-7 |
| A | CN 115270659 A (ELECTRIC POWER SCIENCE RESEARCH INSTITUTE OF STATE GRID ANHUI ELECTRIC POWER CO., LTD.) 01 November 2022 (2022-11-01) entire text, all drawings | 1-7 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 September 2024** | **01 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/029599**

| Patent document cited in search report | Publication date (day/month/year) | Patent family member(s) | Publication date (day/month/year) |
|---|---|---|---|
| CN 115544853 A | 30 December 2022 | (Family: none) | |
| CN 115270659 A | 01 November 2022 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3309820 B
- JP 2023137135 A